# FASCICULE DE BREVET EUROPEEN

(11) **EP 1 390 138 B1**
(45) Date de publication et mention de la délivrance du brevet: **09.08.2006**
(21) Numéro de dépôt: 02738248.0
(22) Date de dépôt: 16.05.2002
(51) Int. Cl.: B01J 13/10, B01J 13/20, A61K 8/11, A61K 9/50, A23P 1/04

(54) **MICROCAPSULES A BASE DE PROTEINES VEGETALES**
MIKROKAPSELN AUS PFLANZLICHEN PROTEINEN
VEGETABLE PROTEIN-BASED MICROCAPSULES

(30) Priorité: 16.05.2001 FR 0106441
(43) Date de publication de la demande: 25.02.2004
(73) Titulaire: MAINELAB, 49100 Angers (FR)
(72) Inventeur: RICHARD, Jo¬l, F-49160 Blou (FR); MORTEAU, Sophie, F-49800 La Bohalle (FR)
(74) Mandataire: Ahner, Francis
(86) Numéro de dépôt international: PCT/FR2002/001652
(87) Numéro de publication internationale: WO 2002/092217

(56) Documents cités:
- WO-A-99/38945
- US-B1- 6 197 757

## Description

La présente invention a pour objet un procédé de préparation de microparticules à base de protéines végétales, et l'utilisation de ces particules dans les domaines pharmaceutique, vétérinaire, cosmétique, agro-alimentaire, chimique et biomédical.

La microencapsulation regroupe l'ensemble des technologies permettant d'obtenir des particules individualisées dont la taille est comprise entre 1 µm et 1 mm, et conduisant à l'inclusion de substances ou de principes actifs au sein d'un matériau support.

On distingue classiquement deux groupes de microparticules:
- les microcapsules ou systèmes réservoirs: ce sont des particules sphériques constituées d'une enveloppe solide et d'un coeur de matière active liquide, solide ou pâteuse,
- les microsphères ou systèmes matriciels : elles sont constituées d'un réseau continu de matériau enrobant dans lequel est dispersée la substance à encapsuler.

Il existe trois types de procédés d'encapsulation: des procédés physico-chimiques (coacervation simple, coacervation complexe, évaporation de solvant, extraction-évaporation de solvant, fusion à chaud du matériau enrobant), des procédés chimiques (polycondensation interfaciale, polymérisation en milieu dispersé et gélification du matériau enrobant) et des procédés mécaniques (encapsulation en lit d'air fluidisé, par atomisation et par prilling).

La coacervation complexe repose sur le phénomène de désolvatation de macromolécules, l'une chargée positivement et l'autre négativement, aboutissant à la formation de deux phases non miscibles, à partir de solutions aqueuses colloïdales initialement homogènes. Ces deux phases sont :
- le coacervat, riche en polymère et appauvri en eau, qui résulte de la formation de complexes entre les macromolécules chargées positivement et celles chargées négativement,
- le surnageant pauvre en polymère et riche en eau.

L'encapsulation d'une huile par coacervation complexe consiste à émulsionner l'huile dans une solution de deux polymères. La coacervation est induite par ajustement du pH du milieu. Les complexes polymères formés s'adsorbent sur les gouttelettes d'huile et les isolent ainsi du milieu extérieur. La paroi formée est durcie par refroidissement du milieu et réticulée par l'action d'un agent de réticulation.

L'encapsulation d'une substance active offre des avantages importants tels que sa protection vis-à-vis d'agents extérieurs ou sa libération lente, retardée ou différée au site d'utilisation.

Pour des applications dans les domaines pharmaceutique, vétérinaire, cosmétique, agro-alimentaire et biomédical, les matériaux les plus recherchés comme constituants de la paroi sont des substances naturelles, en particulier les protéines ou les polysaccharides, en raison de leur biocompatibilité et de leur biodégradabilité. Parmi ces biopolymères, l'albumine, la gélatine, le collagène et la caséine ont fait l'objet de nombreux travaux.

Ainsi, des capsules d'albumine et d'alginate de sodium ont été préparées par coacervation complexe pour développer un système d'encapsulation de protéines et de polypeptides (Singh et al., J. Pharm. Pharmacol., (1989) **41**, 670-673).

Une autre étude décrit l'encapsulation d'un principe actif dans des microsphères de caséine, préparées par émulsion-extraction de solvant, et il a été démontré que cette protéine de lait constituait un support potentiel pour des préparations orales à libération prolongée (Latha et al., J. Control. Rel., (1995) **34**, 1-7).

Depuis quelques années, une nouvelle approche consiste à utiliser des protéines d'origine végétale plutôt qu'animale. En effet, depuis la découverte de l'encéphalopathie spongiforme d'origine animale (maladie de la «vache folle»), les consommateurs ne font plus confiance aux produits susceptibles d'être contaminés par le prion, agent potentiellement responsable de cette pathologie. Il devient donc nécessaire de trouver un substitut aux protéines animales telles que la gélatine et l'albumine. Dans la littérature, plusieurs techniques d'encapsulation à partir de ces polymères d'origine végétale ont été décrites.

Un antibiotique a pu être encapsulé par coacervation simple en utilisant le gluten de blé et la caséine comme matériaux d'enrobage, dans le but d'obtenir un système à libération prolongée (Jiunn-Yann Yu et al., J. of Fermentation and Bioengineering, (1997) Vol **84**, 5, 444-448).

Un autre système à libération contrôlée a été développé à partir de nanoparticules de gliadine (fraction protéique du gluten de blé) obtenues par une méthode basée sur la désolvatation de macromolécules, par addition d'une phase organique de protéine à une phase aqueuse (Ezpeleta et al., Int. J. Pharm., (1996) **131**, 191-200).

D'autres travaux ont montré qu'il était possible de fabriquer des nano- et des microparticules à partir de viciline (protéine de pois) par coacervation simple (Ezpeleta et al., J. Microencapsulation, (1997), Vol. **14**, n° 5, 557-565).

Enfin, il est possible de préparer des particules possédant une paroi formée de protéines végétales par une réaction de réticulation interfaciale entre ces protéines et un agent réticulant polyfonctionnel acylant. Cette méthode permet d'encapsuler des substances actives à l'état de solution, de suspension ou d'émulsion et toute protéine végétale peut être utilisée, notamment celles qui sont extraites du blé, du soja, du pois, du colza, du tournesol, de l'orge ou de l'avoine (WO 99/03450).

En ce qui concerne la méthode de coacervation complexe, les couples classiquement utilisés sont la gélatine comme polycation et l'alginate de sodium, le polyphosphate ou la gomme arabique comme polyanion. Des études ont montré que la gélatine pouvait être substituée par de l'albumine bovine (Singh et al., J. Pharm. Pharmacol., (1989) **41**, 670-673).

Bien que la tendance soit d'utiliser des produits naturels d'origine végétale comme substitut des protéines animales, aucun procédé d'encapsulation par coacervation complexe à partir de protéines végétales n'a pu être mis en oeuvre. En effet, les protéines végétales ne sont pas pures ; elles présentent de gros problèmes de solubilité du fait de la présence d'une fraction soluble et d'une fraction insoluble et possèdent également un pouvoir émulsifiant faible par rapport à celui des protéines animales, ce qui rend nécessaire l'utilisation de tensioactifs supplémentaires qui interfèrent dans la phase de fixation des coacervats.

Or, les inventeurs, de manière surprenante, ont mis au point un procédé qui résout l'ensemble de ces problèmes.

Les inventeurs ont réussi à définir un nouveau procédé qui permet l'utilisation de ces protéines dans une technique de coacervation complexe.

Aussi, la présente invention a pour objet un procédé de fabrication de microcapsules contenant un matériau à encapsuler, caractérisé en ce qu'on soumet un mélange d'au moins une protéine végétale solubilisée et d'un polyélectrolyte de charge opposée à ladite protéine à une coacervation complexe en milieu aqueux suivie éventuellement d'un durcissement, en présence dudit matériau à encapsuler.

Dans un mode de réalisation préférée de l'invention, le procédé comprend:
a) la solubilisation d'au moins une protéine végétale en milieu aqueux à un pH compris entre 2 et 7 et inférieur au pH isoélectrique de ladite protéine,
b) la centrifugation de la solution obtenue en a),
c) le mélange du surnageant obtenu en b) avec une solution aqueuse d'un polyélectrolyte de charge opposée à celle de la protéine végétale,
d) la coacervation des polyélectrolytes sous la forme de complexes de polymères, et éventuellement le durcissement des capsules, en présence du matériau à encapsuler.

L'étape b) de centrifugation est réalisée dans des conditions bien choisies, notamment à une vitesse comprise entre 2 000 et 15 000 t/min préférentiellement entre 4 000 et 12 000 t/min, pendant 10 à 30 minutes.

Dans un mode de réalisation particulièrement avantageux de l'invention, le procédé est caractérisé en ce que l'on augmente la quantité de protéines solubles par:
e) addition d'une quantité de protéines végétales au surnageant dans le but d'atteindre la saturation,
f) centrifugation du mélange, et
g) éventuellement répétition des étapes e) et f) plusieurs fois.

De manière très avantageuse, le procédé de fabrication selon l'invention est caractérisé en ce que l'étape c) est mise en oeuvre à un pH inférieur au pH isoélectrique de la protéine végétale afin que ladite protéine soit utilisée dans l'étape d) de coacervation complexe comme polyélectrolyte cationique.

D'une autre manière avantageuse, le procédé de fabrication d'une paroi de microcapsules à base de protéines végétales est caractérisé en ce que l'étape c) est mise en oeuvre à un pH supérieur au pH isoélectrique de la protéine végétale afin que ladite protéine soit utilisée dans l'étape d) de coacervation complexe comme polyélectrolyte anionique.

La concentration en protéines dans la solution initiale est généralement comprise entre 2 et 15%, la concentration du surnageant de la solution initiale de protéines centrifugées entre 1 et 8%, et la concentration en protéines dans la solution de coacervation entre 1,5 et 5%.

Les protéines végétales utilisées dans le cadre de l'invention sont extraites de végétaux choisis dans le groupe comprenant: lupin (genre Lupinus), soja (genre Glycine), pois (genre Pisum), pois chiche (Cicer), luzerne (Medicago), féverole (Vicia), lentille (Lens), haricot (Phaseolus), colza (Brassica), tournesol (Helianthus) et des céréales comme le blé, le maïs, l'orge, le malt et l'avoine. A titre d'exemple, on peut citer les protéines végétales SWP100 et SWP50 et celles commercialisées sous le nom Supro® 670 et PISANE® .

Avantageusement, le polyélectrolyte anionique est choisi parmi ceux classiquement utilisés par l'homme du métier, notamment ceux qui sont choisis dans le groupe comprenant l'alginate de sodium, la gomme arabique, les polyphosphates, la carboxyméthylcellulose de sodium, le carraghénanne, la gomme xanthane et les protéines végétales de pH supérieur au pHi. De manière avantageuse, le polyélectrolyte cationique est un de ceux classiquement utilisés par l'homme du métier notamment ceux qui sont choisis dans le groupe comprenant les agents tensioactifs cationiques, les latex possédant un ammonium quaternaire, le chitosane et les protéines végétales de pH inférieur au pHi.

Lorsque le procédé comprend une étape de durcissement, celle-ci peut être réalisée par toute technique connue de l'homme du métier notamment par réticulation par un agent réticulant choisi dans le groupe comprenant les dialdéhydes tels que le glutaraldéhyde et les tanins tels que l'acide tannique.

Lorsque le polyélectrolyte cationique est le chitosane, le durcissement est réalisé en utilisant l'anhydride acétique comme agent durcisseur.

Dans un mode de réalisation particulièrement avantageux de l'invention, on utilise un couple polycation/polyanion choisi dans le groupe comprenant les couples: SWP100/alginate, SWP100/gomme arabique, chitosane/Supro® , Supro® /alginate ou Supro® /gomme arabique.

Les microcapsules obtenues par le procédé selon l'invention ont un diamètre compris entre 5 et 500 µm, de préférence 20 et 200 µm, plus préférentiellement de 20 à 50 µm.

Les microcapsules selon l'invention peuvent contenir des substances aptes à être utilisées dans les domaines pharmaceutique, vétérinaire, cosmétique, agro-alimentaire, chimique et biomédical, en particulier des principes actifs. Elles peuvent être combinées avec tout ingrédient actif ou tout excipient bien connu de l'homme du métier.

Les exemples qui suivent illustrent l'invention sans toutefois la limiter.

### EXEMPLE 1 : Coacervation complexe à partir de la protéine SWP100 en présence d'alginate.

Une solution de SWP100 à 10% maintenue à pH 3 est centrifugée pendant 25 minutes à 4500 t/min. On obtient 48 mL de surnageant contenant 0,72 g de protéines dissoutes. Dans cette solution de surnageant, 20 g de Miglyol® 812 sont émulsionnés. On ajoute alors 35,6 mL d'une solution aqueuse d'alginate de sodium (0,36 g) et ensuite un volume de 96 mL d'eau. La température du milieu est de 40 °C. Le pH du milieu est abaissé de 4,22 à 3 par ajout d'acide chlorhydrique 1N.

Le rapport massique SWP100/alginate est égal à 2 et la concentration finale dans la phase aqueuse en SWP100 est de 0,4% poids/volume et elle est de 0,2% poids/volume pour l'alginate.

La coacervation complexe se réalise et le milieu est refroidi à 10 °C et maintenu à 10°C pendant 1 heure. 1,5 mL de glutaraldéhyde à 25% est additionné dans le milieu à 10°C. Puis on laisse revenir le milieu à température ambiante et le milieu est maintenu sous agitation pendant 15 heures.

On obtient une dispersion de microcapsules contenant 95% d'huile, de taille moyenne comprise entre 200 et 400 µm.

Des microcapsules dont le rapport massique SWP100/alginate est égal à 1, sont préparées par la même technique.

### EXEMPLE 2 : Coacervation complexe à partir de la protéine SWP100 en présence de gomme arabique.

Une solution de 100 mL de SWP100 à 17% maintenue à pH 3 est centrifugée pendant 25 minutes à 4500 t/min. On obtient 100 mL de surnageant contenant 2,6 g de protéines dissoutes. Dans cette solution de surnageant, 20 g de Miglyol® 812 sont émulsionnés. On ajoute 45 mL d'une solution aqueuse de gomme arabique (5 g) et un volume d'eau de 13 mL. La température du milieu est de 40°C. Le pH du milieu est abaissé à 3 par ajout d'acide chlorhydrique 1 N.

Le rapport massique SWP100/gomme arabique est égal à 1/2 et la concentration finale de SWP100 dans la phase aqueuse est de 1,5% poids/volume et elle est de 3% poids/volume pour la gomme arabique.

La coacervation complexe se réalise et le milieu est refroidi à 10 °C. Le milieu est laissé sous agitation pendant 1 heure et 3 mL de glutaraldéhyde à 25% sont ensuite ajoutés. Puis on laisse revenir le milieu à température ambiante toujours sous agitation pendant 6 heures.

On obtient une dispersion de microcapsules contenant 72,5% d'huile, de taille moyenne comprise entre 150 et 450 µm.

### EXEMPLE 3 : Influence de l'augmentation de la concentration en protéine SWP100 dans le surnageant. Coacervation complexe à partir de la protéine SWP100 en présence d'alginate.

Le pH d'une solution protéique de SWP 100 à environ 15% (20 g dans 130 g d'eau) est ajusté à un valeur de 4. La solution est centrifugée une première fois à 12 000 t/min pendant 15 minutes. Le culot est retiré et une quantité de 12 g de protéines SWP100 est ajoutée dans le surnageant dont le pH est à nouveau ajusté à 4.

Une seconde centrifugation est effectuée et cette opération est répétée une troisième fois.

Après la première centrifugation, le surnageant contient 2,9% de protéine soluble. Après trois centrifugations, la concentration en protéine soluble est de 3,6%.

Le rapport massique SWP100/gomme arabique est égal à 1 et la concentration finale de SWP100 et de gomme arabique dans la phase aqueuse est de 2% poids/volume.

La coacervation complexe est réalisée avec le surnageant concentré de SWP100 à pH 4 (100 mL contenant 3, 6 g de protéine) et la gomme arabique comme polyélectrolyte anionique (80 mL contenant 3,6 g de gomme arabique). La coacervation est réalisée selon le mode opératoire décrit dans l'exemple 1.

On obtient une dispersion de microcapsules contenant 73,5 % d'huile, de taille moyenne comprise entre 50 et 400 µm.

### EXEMPLE 4 : Coacervation complexe à partir du couple Supro® 670/alginate

Des capsules sont préparées selon le mode opératoire de l'exemple 1 à partir d'une solution de Supro® 670 composée de 22,5 g d'eau et 2,5 g de protéine et une solution d'alginate composée de 150 g d'eau et 1,84 g d'alginate.

Le pH de coacervation est égal à 3,8.

On obtient des microcapsules en suspension qui contiennent 82 % d'huile et qui présentent une paroi fragile à l'aspect granuleux. La taille moyenne des microcapsules est comprise entre 50 et 400 µm.

### EXEMPLE 5 : Evaluation des protéines végétales comme polyélectrolyte anionique

Une solution de protéine Supro® 670 à 10% est ajustée à pH 7, et centrifugée une première fois à 4500 t/min pendant 25 minutes. Le culot est retiré et le surnageant composé de 43 mL d'eau et 2,57 g de protéine est utilisé pour la coacervation.

20 g de Miglyol® 812 sont émulsionnés dans le surnageant de la solution de protéine Supro® 670, puis une solution de chitosane 2622 composée de 120 mL d'eau et 1,5 g de chitosane à pH 1,32 est ajouté dans le milieu à 40°C.

Le pH de coacervation est ajusté à 6. Le mode opératoire est ensuite identique à celui décrit pour l'exemple 1.

On obtient une dispersion de microcapsules contenant 83 % d'huile, de taille moyenne comprise entre 50 et 400 µm.

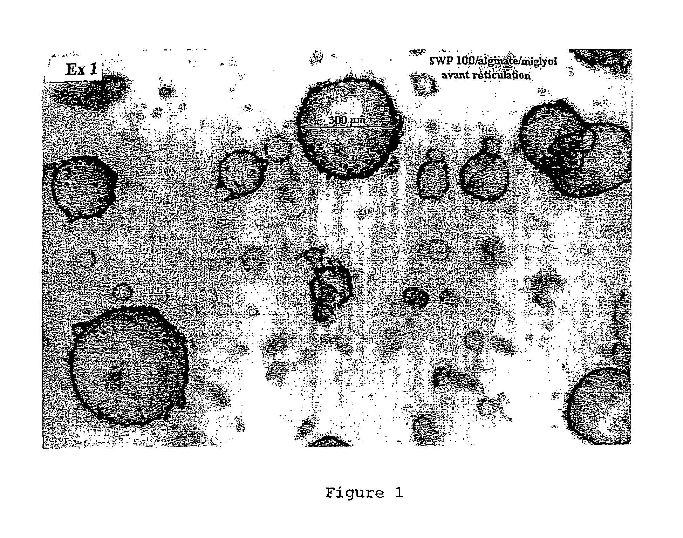

## Revendications

1. Procédé de fabrication de microcapsules contenant un matériau à encapsuler, **caractérisé en ce qu'**on soumet un mélange d'au moins une protéine végétale solubilisée et d'un polyélectrolyte de charge opposée à ladite protéine à une coacervation complexe en milieu aqueux suivie éventuellement d'un durcissement, en présence dudit matériau à encapsuler.

2. Procédé de fabrication de microcapsules à base de protéines végétales selon la revendication 1, **caractérisé en ce qu'**il comprend
a) la solubilisation d'au moins une protéine végétale en milieu aqueux à un pH compris entre 2 et 7 et inférieur au pH isoélectrique de ladite protéine,
b) la centrifugation de la solution obtenue en a),
c) le mélange du surnageant obtenu en b) avec une solution aqueuse d'un polyélectrolyte de charge opposée à celle de la protéine végétale,
d) la coacervation des polyélectrolytes sous la forme d'un complexe de polymères, et éventuellement le durcissement des capsules, en présence du matériau à encapsuler.

3. Procédé de fabrication de microcapsules à base de protéines végétales selon la revendication 2, **caractérisé en ce que** l'on augmente la quantité de protéines solubles par :
e) addition d'une quantité de protéines végétales au surnageant obtenue à l'étape b),
f) centrifugation du mélange et
g) éventuellement répétition des étapes e) et f) plusieurs fois.

4. Procédé de fabrication de microcapsules à base de protéines végétales selon l'une quelconque des revendications 1 à 3, **caractérisé en ce que** l'étape c) est mise en oeuvre à un pH inférieur au point isoélectrique de la protéine végétale afin que ladite protéine soit utilisée dans l'étape d) de coacervation complexe comme polyélectrolyte cationique.

5. Procédé de fabrication de microcapsules à base de protéines végétales selon l'une quelconque des revendications 1 à 3, **caractérisé en ce que** l'étape c) est mise en oeuvre à un pH supérieur au pH isoélectrique de la protéine végétale afin que ladite protéine soit utilisée dans l'étape d) de coacervation complexe comme polyélectrolyte anionique.

6. Procédé selon l'une quelconque des revendications 1 à 5, **caractérisé en ce que** les protéines végétales sont extraites de végétaux choisis dans le groupe comprenant lupin (genre Lupinus), soja (genre Glycine), pois (genre Pisum), pois chiche (Cicer), luzerne (Medicago), féverole (Vicia), lentille (Lens), haricot (Phaseolus), colza (Brassica), tournesol (Helianthus) et des céréales comme le blé, le maïs, l'orge, le malt et l'avoine.

7. Procédé selon l'une quelconque des revendications 1 à 4 et 6, **caractérisé en ce que** le polyélectrolyte anionique est choisi dans le groupe comprenant l'alginate de sodium, la gomme arabique, les polyphospahtes, la carboxyméthylcellulose de sodium, le carraghénanne, la gomme xanthane et les protéines végétales de pH supérieur au pHi.

8. Procédé selon l'une quelconque des revendications 1 à 3 et 5 à 6, **caractérisé en ce que** le polyélectrolyte cationique est choisi dans le groupe comprenant des agents tensioactifs cationiques, des latex possédant un ammonium quaternaire, le chitosane et les protéines végétales de pH inférieur au pHi.

9. Procédé selon l'une quelconque des revendications 1 à 7, **caractérisé en ce que** le durcissement est réalisé par réticulation par un agent réticulant choisi dans le groupe comprenant les dialdéhydes tels que le glutaraldéhyde et les tanins tels que l'acide tannique.

10. Procédé selon la revendication 8, **caractérisé en ce que** lorsque le polyélectrolyte cationique est le chitosane, le durcissement est réalisé en utilisant l'anhydride acétique comme agent durcisseur.

11. Procédé selon l'une quelconque des revendications 1 à 10, **caractérisé en ce que** l'on utilise un couple polycation/polyanion choisi dans le groupe comprenant les couples: SWP100/alginate, SWP100/gomme arabique, chitosane/Supro® , Supro® /alginate ou Supro® /gomme arabique.

12. Utilisation des capsules obtenues par le procédé selon l'une quelconque des revendications 1 à 10 pour la fabrication de compositions utiles dans les domaines pharmaceutique, vétérinaire, cosmétique, agro-alimentaire, chimique et biomédical.

13. Compositions pharmaceutique, vétérinaire, cosmétique, agro-alimentaire ou chimique, **caractérisées en ce qu'**elles contiennent des microcapsules selon l'une quelconque des revendications 1 à 11, en association avec tout excipient acceptable.

## Claims

1. Method for producing microcapsules containing a material to be encapsulated, **characterized in that** a mixture of at least one solubilized plant protein and a polyelectrolyte with an opposite charge to said protein is subjected to complex coacervation in an aqueous medium, optionally followed by hardening, in the presence of said material to be encapsulated.

2. Method for producing plant protein-based microcapsules according to Claim 1, **characterized in that** it comprises:
a) solubilization of at least one plant protein in an aqueous medium at a pH of between 2 and 7, and below the isoelectric pH of said protein,
b) centrifugation of the solution obtained in a),
c) mixing of the supernatant obtained in b) with an aqueous solution of a polyelectrolyte with an opposite charge to that of the plant protein,
d) coacervation of the polyelectrolytes in the form of a polymeric complex, and optionally hardening of the capsules, in the presence of the material to be encapsulated.

3. Method for producing plant protein-based microcapsules according to Claim 2, **characterized in that** the amount of soluble proteins is increased by:
e) addition of an amount of plant proteins to the supernatant obtained in step b),
f) centrifugation of the mixture, and
g) optionally repetition of steps e) and f) several times.

4. Method for producing plant protein-based microcapsules according to any one of Claims 1 to 3, **characterized in that** step c) is carried out at a pH below the isoelectric pH of the plant protein, so that said protein is used as a cationic polyelectrolyte in the complex coacervation step d).

5. Method for producing plant protein-based microcapsules according to any one of Claims 1 to 3, **characterized in that** step c) is carried out at a pH above the isoelectric pH of the plant protein, so that said protein is used as an anionic polyelectrolyte in the complex coacervation step d).

6. Method according to any one of Claims 1 to 5, **characterized in that** the plant proteins are extracted from plants chosen from the group comprising: lupin (genus Lupinus), soybean (genus Glycine), pea (genus Pisum), chickpea (Cicer), alfalfa (Medicago), broad bean (Vicia), lentil (Lens), bean (Phaseolus), rapeseed (Brassica), sunflower (Helianthus) and cereals such as wheat, maize, barley, malt or oats.

7. Method according to any one of Claims 1 to 4 and 6, **characterized in that** the anionic polyelectrolyte is chosen from the group comprising sodium alginate, gum arabic, polyphosphates, sodium carboxymethylcellulose, carrageenan, xanthan gum and plant proteins with a pH above the pHi.

8. Method according to any one of Claims 1 to 3 and 5 to 6, **characterized in that** the cationic polyelectrolyte is chosen from the group comprising cationic surfactants, latexes having a quaternary ammonium, chitosan and plant proteins with a pH below the pHi.

9. Method according to any one of Claims 1 to 7, **characterized in that** the hardening is carried out by crosslinking with a crosslinking agent chosen from the group comprising dialdehydes such as glutaraldehyde and tannins such as tannic acid.

10. Method according to Claim 8, **characterized in that**, when the cationic polyelectrolyte is chitosan, the hardening is carried out using acetic anhydride as hardening agent.

11. Method according to any one of Claims 1 to 10, **characterized in that** use is made of a polycation/polyanion couple chosen from the group comprising the couples: SWP100/alginate, SWP100/gum arabic, chitosan/Supro®, Supro®/alginate or Supro®/gum arabic.

12. Use of the capsules obtained by the method according to any one of Claims 1 to 10, for producing compositions which are useful in the pharmaceutical, veterinary, cosmetics, agrofoods, chemical and biomedical fields.

13. Pharmaceutical, veterinary, cosmetic, agrofoods or chemical composition, **characterized in that** it contains microcapsules according to any one of Claims 1 to 11, in combination with any acceptable excipient.

## Patentansprüche

1. Verfahren zur Herstellung von Mikrokapseln, enthaltend eine einzukapselnde Substanz, **dadurch gekennzeichnet, dass** man eine Mischung aus mindestens einem solubilisierten pflanzlichen Protein und einem Polyelektrolyt, mit einer dem Protein entgegengesetzten Ladung einer komplexen Koazervation in einem wässrigen Medium unterzieht, gegebenenfalls gefolgt von einer Härtung in Gegenwart der einzukapselnden Substanz.

2. Verfahren zur Herstellung von Mikrokapseln auf der Grundlage von pflanzlichen Proteinen gemäß Anspruch 1, **dadurch gekennzeichnet, dass** es umfasst:
(a) Solubilisierung mindestens eines pflanzlichen Proteins in wässrigem Medium mit einem pH-Wert im Bereich von 2 bis 7 und unter dem isoelektrischen pH-Wert des Proteins,
(b) Zentrifugieren der in a) erhaltenen Lösung,
(c) Mischen des in b) erhaltenen Überstandes mit einer wässrigen Lösung eines Polyelektrolyts mit einer Ladung, die der Ladung des pflanzlichen Proteins entgegengesetzt ist,
(d) Koazervation des Polyelektrolyten in Form eines Polymerkomplexes und gegebenenfalls Härten der Kapseln in Gegenwart der einzukapselnden Substanz.

3. Verfahren zur Herstellung von Mikrokapseln auf der Grundlage von pflanzlichen Proteinen nach Anspruch 2, **dadurch gekennzeichnet, dass** man die Menge der löslichen Proteine erhöht durch:
e) Zugabe einer Menge der pflanzlichen Proteine zum in Schritt b) erhaltenen Überstand,
f) Zentrifugieren der Mischung und
g) gegebenenfalls mehrmaliges Wiederholen der Schritte e) und f).

4. Verfahren zur Herstellung von Mikrokapseln auf der Grundlage von pflanzlichen Proteinen gemäß einem der Ansprüche 1 bis 3, **dadurch gekennzeichnet, dass** Schritt c) bei einem pH-Wert ausgeführt wird, der unter dem isoelektrischen Punkt des pflanzlichen Proteins liegt, damit das Protein in Schritt d) der komplexen Koazervation als kationisches Polyelektrolyt verwendet werden kann.

5. Verfahren zur Herstellung von Mikrokapseln auf der Grundlage von pflanzlichen Proteinen gemäß einem der Ansprüche 1 bis 3, **dadurch gekennzeichnet, dass** Schritt c) bei einem pH-Wert ausgeführt wird, der über dem isoelektrischen pH-Wert des pflanzlichen Proteins liegt, damit das Protein in Schritt d) der komplexen Koazervation als anionisches Polyelektrolyt verwendet werden kann.

6. Verfahren gemäß einem der Ansprüche 1 bis 5, **dadurch gekennzeichnet, dass** die pflanzlichen Proteine pflanzliche Extrakte sind, ausgewählt aus der Gruppe umfassend: Lupine (Gattung Lupinus), Soja (Gattung Glyzin), Erbsen (Gattung Pisum), Kichererbse (Cicer), Luzerne (Medicago), Ackerbohne (Vicia), Linse (Lens), Bohne (Phaseolus), Raps (Brassica), Sonnenblume (Helianthus) und Getreide wie Weizen, Mais, Gerste, Malz und Hafer.

7. Verfahren gemäß einem der Ansprüche 1 bis 4 und 6, **dadurch gekennzeichnet, dass** der anionische Polyelektrolyt ausgewählt ist aus der Gruppe umfassend Natriumalginat, Gummiarabicum, Polyphosphate, Natriumcarboxymethylcellulose, Carrageenan, Xanthangummi und pflanzliche Proteine, deren pH-Wert höher ist als der pHi.

8. Verfahren gemäß einem der Ansprüche 1 bis 3 und 5 bis 6, **dadurch gekennzeichnet, dass** der kationische Polyelektrolyt ausgewählt ist aus der Gruppe umfassend: kationische, tensioaktive Agenzien, Latex mit einem quaternären Ammonium, Chitosan und pflanzliche Proteine mit einem pH-Wert, der niedriger ist als der pHi.

9. Verfahren gemäß einem der Ansprüche 1 bis 7, **dadurch gekennzeichnet, dass** die Härtung durch Vernetzung mit einem Vernetzungsmittel erfolgt, das ausgewählt ist aus der Gruppe umfassend Dialdehyde wie Glutaraldehyd und Tannine wie Gerbsäure.

10. Verfahren gemäß Anspruch 8, **dadurch gekennzeichnet, dass**, wenn der kationische Polyelektrolyt Chitosan ist, die Härtung unter Verwendung eines Acetanhydrids als Härtungsmittel erfolgt.

11. Verfahren gemäß einem der Ansprüche 1 bis 10, **dadurch gekennzeichnet, dass** ein Polykation/Polyanion-Paar, ausgewählt aus der Gruppe umfassend die Paare: SWP100/Alginat, SWP100/Gummiarabicum, Chitosan/Supro® , Supro® /Alginat oder Supro® /Gummiarabicum, verwendet wird.

12. Verwendung der im Verfahren gemäß einem der Ansprüche 1 bis 10 erhaltenen Kapseln zur Herstellung von Zusammensetzungen, die im Bereich Pharmazie, Tiermedizin, Kosmetik, Lebensmittel, Chemie und Biomedizin nützlich sind.

13. Pharmazeutische, veterinärmedizinische, kosmetische Zusammensetzungen, Zusammensetzungen aus dem Lebensmittelbereich oder chemische Zusammensetzungen, **dadurch gekennzeichnet, dass** sie Mikrokapseln gemäß einem der Ansprüche 1 bis 11 zusammen mit jedem geeigneten Exzipienten enthalten.
